# FASCICULE DE BREVET EUROPEEN

(11) **EP 4 288 410 B1**
(45) Date de publication et mention de la délivrance du brevet: **29.01.2025**
(21) Numéro de dépôt: 22708580.0
(22) Date de dépôt: 31.01.2022
(51) Int. Cl.: C07C 227/08, C07C 229/08

(54) **PROCEDE D'AMMONOLYSE D'ACIDES BROMOALCANOIQUES**
VERFAHREN ZUR AMMONOLYSE VON BROMALKANSÄUREN
METHOD FOR AMMONOLYSIS OF BROMOALKANOIC ACIDS

(30) Priorité: 02.02.2021 FR 2100997
(43) Date de publication de la demande: 13.12.2023
(73) Titulaire: ARKEMA FRANCE, 92700 Colombes (FR)
(72) Inventeur: DEVAUX, Jean-François, 69491 PIERRE-BENITE CEDEX (FR); PEES, Bernard, 13374 MARSEILLE CEDEX 11 (FR)
(74) Mandataire: Arkema Patent
(86) Numéro de dépôt international: PCT/FR2022/050165
(87) Numéro de publication internationale: WO 2022/167748

(56) Documents cités:
- FR-A- 928 265

## Description

### [Domaine technique]

La présente demande de brevet concerne un procédé d'ammonolyse d'acides ω-bromoalcanoïques pour fabriquer les acides ω-aminoalcanoïques correspondants, utiles comme monomères de polyamides.

### [Technique antérieure]

Des procédés d'ammonolyse d'acides ω-bromoalcanoïques de formule Br-(CH₂)ₙ-COOH n=9 à 11 en acide ω-aminoalcanoïque NH₂-(CH₂)ₙ-CO₂H en utilisant de l'ammoniac sont connus.

Le document FR 988699 décrit ainsi la réaction de l'acide 10-bromodécanoïque avec une solution aqueuse d'ammoniac à 25% à 15°C pendant 6 jours pour former de l'acide 10-aminodécanoïque avec un rendement de 77%. Ce procédé présente comme inconvénient un temps de réaction très long.

L'augmentation de la température permet d'accélérer la réaction, mais favorise les réactions secondaires.

Ainsi, dans le document FR 928265, on rapporte la réaction de l'acide 11-bromoundécanoïque réalisée à 60°C avec une solution aqueuse d'ammoniac à 25% pendant 10 heures en récipient clos, et donc sous pression autogène. Le rendement de cette réaction est de 53% et donc très faible.

En effet, lorsqu'on opère l'ammonolyse, on coproduit notamment de l'amine secondaire de formule HO₂C-(CH₂)ₙ-NH-(CH₂)ₙ-CO₂H en quantité plus ou moins importante. Cette impureté est gênante dans l'utilisation des acides ω-aminoalcanoïques pour fabriquer des polyamides, car elle induit des branchements de chaînes. Comme cette amine secondaire diminue le rendement et est difficile à séparer de l'amine primaire souhaitée, on cherche à minimiser sa formation.

La demande de brevet CN 103804209 B décrit à l'exemple 3 l'ammonolyse d'acide 11-bromoundécanoïque avec de l'ammoniac anhydre sous pression de 0.1 à 0.15 MPa en présence de solvant et d'un catalyseur de transfert de phase. Ce procédé permet de réduire le temps de réaction à moins de 24 heures, mais fonctionne en milieu très dilué, et requiert donc des installations industrielles de taille importante, et présente un rendement encore assez modeste, de 84.3%.

La demande de brevet EP 2 358 662 B1 propose de diminuer le temps de réaction de l'ammonolyse de l'acide 11-bromoundécanoïque ainsi que les réactions secondaires en conduisant la réaction avec un profil de température croissante, le milieu réactionnel étant soumis à une montée en température par paliers réguliers entre une température initiale de 15 à 25°C et une température finale de 26 à 40°C.

On est toujours à la recherche de solutions techniques permettant de minimiser la production d'amine secondaire tout en maîtrisant les tailles d'installation.

### [Résumé de l'invention]

L'invention a pour but de proposer une solution à un ou plusieurs des problèmes évoqués ci-dessus.

En effet, la présente invention repose sur la constatation inattendue que l'augmentation du titre de la solution aqueuse d'ammoniac au-dessus de 35% massique, associée à une augmentation de la pression au-dessus de la pression atmosphérique permet d'accélérer la réaction tout en maîtrisant la quantité d'amine secondaire produite et donc d'augmenter le rendement.

Aussi, selon un premier aspect, l'invention a pour objet un procédé de fabrication d'acide ω-aminoalcanoïque de formule NH₂-(CH₂)ₙ-COOH, dans laquelle n est un entier de 9 à 11, par réaction de l'acide bromoalcanoïque correspondant avec l'ammoniac, comprenant les étapes suivantes:
i) réaction de l'acide ω-bromoalcanoïque avec une solution aqueuse d'ammoniac en excès,
ii) séparation de l'acide ω-aminoalcanoïque formé du mélange réactionnel,
caractérisé en ce que la solution aqueuse d'ammoniac présente une concentration de 35 à 70% massique et que l'étape i) est conduite sous une pression supérieure à la pression atmosphérique, de 0.11 à 2.0 MPa absolu.

De préférence, l'étape i) est réalisée à une température comprise entre 0 et 60°C. Elle peut être réalisée à une température constante ou à une température croissante. Lorsqu'elle est réalisée à une température croissante, l'étape i) peut avantageusement être réalisée avec une température initiale allant de 0 à 25°C et une température finale allant de 26 à 60°C.

De préférence, la solution aqueuse d'ammoniac présente une concentration de 36 à 60%, de préférence 38 à 45% massique.

Avantageusement, le ratio massique entre l'acide ω-bromoalcanoïque au début de l'étape i) et la solution aqueuse d'ammoniac est de 1:3 à 1:20.

L'étape i) du procédé selon l'invention peut être conduite notamment sous une pression de 0.125 à 0.5 MPa absolu, ou encore de 0.13 à 0.3 MPa absolu.

Le procédé de l'invention peut être réalisé en mode continu ou en mode batch.

Avantageusement, le procédé selon l'invention comprend en outre une étape iii) de lavage et essorage de l'acide ω-aminoalcanoïque obtenu à l'issue de l'étape ii).

Il peut également comprendre en outre une étape iv) de purification de l'acide ω-aminoalcanoïque brut obtenu à l'issue de l'étape ii ou iii), de préférence par recristallisation dans l'eau bouillante.

Il peut par ailleurs aussi comprendre en outre une étape v) de récupération de l'acide ω-aminoalcanoïque résiduel dans les différents filtrats et eaux de lavage obtenues dans les étapes ii), iii) et/ou iv), notamment par dégazage de l'ammoniac, extraction liquide/liquide, cristallisation, filtration et lavage.

Enfin, le procédé de l'invention peut comprendre en outre une étape vi) de séchage de l'acide ω-aminoalcanoïque obtenu.

### [Description des modes de réalisation]

### Définition des termes

Dans l'ensemble de cet exposé, les pourcentages sont, sauf mention contraire, entendu comme étant des pourcentages massiques par rapport à la composition considérée.

Par ailleurs, les valeurs de pression données dans le présent exposé sont, sauf mention contraire, entendues comme étant des valeurs de pression absolue, c'est-à-dire en référence au vide absolu.

On entend par la terme « solution aqueuse d'ammoniac » une solution de NH₃ dans l'eau. Sa teneur en ammoniac est exprimée en masse de NH₃ par rapport à la masse de solution (NH3 et eau).

Selon la présente invention, le procédé de fabrication d'acide ω-aminoalcanoïque de formule NH₂-(CH₂)ₙ-COOH, dans laquelle n est un entier de 9 à 11, par réaction de l'acide ω-bromoalcanoïque correspondant avec l'ammoniac, comprend donc les étapes suivantes:
i) réaction de l'acide ω-bromoalcanoïque avec une solution aqueuse d'ammoniac en excès,
ii) séparation de l'acide w-aminoalcanoïque formé du mélange réactionnel,
caractérisé en ce que la solution aqueuse d'ammoniac présente une concentration de 35 à 70% massique et que l'étape i) est conduite sous une pression supérieure à la pression atmosphérique, de 0.11 à 2.0 MPa absolu. L'acide ω-bromoalcanoïque peut être l'acide 10-bromodécanoïque, l'acide 11-bromoundécanoïque ou l'acide 12-bromododécanoïque.

Ces acides ω-bromoalcanoïques peuvent être obtenus notamment par réaction d'hydrobromuration de l'acide insaturé correspondant, à savoir l'acide 9-décénoïque, 10-undécénoïque ou 11-dodécénoïque. Ces composés sont disponibles dans le commerce. L'acide 9-décénoïque peut par ailleurs être obtenu à partir d'huile végétale à base oléique selon le procédé décrit dans WO 2018/080869. L'acide 10-undécénoïque peut être obtenu à partir d'huile de ricin selon FR 928265.

On désigne sous le nom d'acide ω-bromoalcanoïque ledit composé, sous forme plus ou moins purifiée, donc y compris ses impuretés éventuelles. L'acide ω-bromoalcanoïque commercial a généralement une pureté supérieure à 98%. Néanmoins, selon un mode de réalisation, la pureté de l'acide ω-bromoalcanoïque mis en oeuvre peut être aussi comprise entre 90% et 98% et de préférence entre à 93% et 96%. L'acide ω-bromoalcanoïque peut contenir comme impureté notamment l'acide bromoalcanoïque de formule CH₃-CH₂Br-(CH₂)ₙ₋₂-CO₂H.

Le procédé de l'invention comporte tout d'abord une étape i) dans laquelle l'on mélange l'acide ω-bromoalcanoïque avec la solution aqueuse d'ammoniac en excès puis on la fait réagir.

L'acide ω-bromoalcanoïque peut être ajouté sous forme solide ou sous forme liquide, notamment à l'état fondu. La forme solide peut être une poudre, des granulés ou des écailles. Avantageusement, l'acide ω-bromoalcanoïque est utilisé à l'état fondu, de préférence à une température comprise de 5 à 60°C, de préférence 10 à 40°C et en particulier 15 à 30°C au-dessus de son point de fusion.

Selon l'invention, la solution aqueuse d'ammoniac utilisée contient de 35 à 70% massique d'ammoniac. De préférence, elle contient 36 à 60% et encore préféré 38 à 45% massique d'ammoniac. Selon un mode de réalisation, la teneur en ammoniac de la solution aqueuse d'ammoniac peut être de 35 à 40%, ou de 40 à 45%, ou de 45 à 50%, ou de 50 à 55%, ou de 55 à 60%, ou de 60 à 65% ou encore de 65 à 70% massique.

Avantageusement, une partie de cette solution aqueuse d'ammoniac provient de la récupération de l'ammoniac en excès et de l'ammonium présents dans le milieu réactionnel en sortie du présent procédé.

Avantageusement, la solution aqueuse d'ammoniac introduite dans le réacteur présente une température qui n'est pas supérieure à 25°C. De préférence, elle est refroidie à une température comprise entre -20°C et 20°C et de préférence une température comprise entre -10 et 10°C.

Le ratio massique entre l'acide ω-bromoalcanoïque et la solution aqueuse d'ammoniac introduit à l'étape i) est tel que l'ammoniac est présent en excès stoechiométrique. Cet excès ne favorise pas seulement la réaction, mais permet également de disperser ou diluer l'acide ω-bromoalcanoïque et le produit formé. Le plus souvent, il est compris entre 1:20 et 1:3, de préférence entre 1:10 et 1:4, et de préférence entre 1:8 et 1:4. On désigne ici par le ratio massique aussi bien le ratio des masses de réactifs utilisés dans le cas d'un procédé batch que le ratio des débits massiques des réactifs dans le cas d'un procédé continu.

Le procédé de l'invention comporte par ailleurs une étape ii) dans laquelle on sépare l'acide ω-aminoalcanoïque formé du mélange réactionnel.

Le mélange réactionnel est le plus souvent non homogène. Il peut comporter notamment une phase gaz, une ou plusieurs phases liquides et/ou une ou plusieurs phases solides.

Le procédé de l'invention peut être conduit en mode continu ou en mode batch.

Lorsque le procédé est mis en oeuvre en mode continu, le mélange des réactifs peut être réalisée dans une cuve munie d'un agitateur. En variante, il peut également être réalisé dans un dispositif de mélange externe comme par exemple un mélangeur statique, un dispositif venturi, ou une boucle de recirculation dans laquelle l'acide ω-bromoalcanoïque est injecté.

L'étape i) du procédé peut être mis en oeuvre dans le même dispositif. Selon un mode de réalisation, l'étape i) peut être réalisée dans une ou plusieurs cuves agitées. Selon un mode de réalisation préféré, l'étape i) est réalisée dans une batterie de 2 à 25 réacteurs connectés en série, dans lequel le mélange réactionnel est envoyé d'un réacteur à l'autre à l'aide d'une pompe ou par écoulement gravitaire. Chaque réacteur peut être constitué d'une cuve munie d'une agitation. Cette agitation peut être réalisée par des modules d'agitation à l'intérieur du réacteur ou par une recirculation externe.

De préférence, l'étape i) est conduite à une température de 0 à 60°C. Selon un mode de réalisation, la température peut être de 0 à 5°C, ou de 5 à 10°C, ou de 10 à 15°C, ou de 15 à 20°C, ou de 20 à 25°C, ou de 25 à 30°C, ou de 30 à 35°C, ou de 35 à 40°C, ou de 40 à 45°C, ou de 45 à 50°C, ou de 50 à 55°C, ou encore de 55 à 60°C.

La température du ou des réacteurs pourra avantageusement être contrôlée par la circulation de fluide caloporteur dans une double enveloppe du ou des réacteurs ou des échangeurs de chaleur dans le ou les réacteurs ou à l'extérieur du ou des réacteurs ou par injection d'un fluide chaud, notamment de l'eau ou de la vapeur, dans un ou plusieurs réacteurs.

La température peut être contrôlée aussi par l'évaporation d'une partie de l'ammoniac afin de refroidir le milieu réactionnel, comme expliqué plus en détail plus loin.

Lorsque le procédé est réalisé dans une batterie de réacteurs comme expliqué ci-dessus, la température dans le premier réacteur est de préférence de 0 à 25°C, et celle dans le dernier réacteur de 26 à 60°C, et la température augmente de réacteur en réacteur. Dans un tel cas, il est avantageux de prévoir un contrôle de la température individuel dans chaque réacteur. Avantageusement, on pourra refroidir le ou les premiers réacteurs et chauffer le ou les derniers réacteurs.

Le temps de séjour total pour l'étape i) (calculé comme le ratio de la somme des volumes de phase liquide dans les réacteurs sur la somme des débits de réactifs) est généralement de 20 à 100 heures et de préférence de 40 à 80 heures.

Selon une deuxième variante de réalisation, l'étape i) du procédé est mis en oeuvre en batch dans un réacteur qui peut être une cuve munie de moyens d'agitation. Selon un mode de réalisation, les réactifs sont mélangés hors du réacteur puis introduits en mélange dans le réacteur. Selon un autre mode de réalisation, la solution d'ammoniac est introduite en premier dans le réacteur puis on ajoute l'acide ω-bromoalcanoïque.

Le mélange de l'acide ω-bromoalcanoïque dans la solution aqueuse d'ammoniac peut être réalisée dans une cuve munie d'un agitateur. En variante, le mélange peut être réalisé au moyen d'un dispositif de mélange externe au réacteur comme par exemple un mélangeur statique ou d'un dispositif venturi permettant de mélanger en ligne les deux flux de réactifs avant leur injection dans le réacteur, ou encore d'une boucle de recirculation externe au réacteur dans laquelle l'acide ω-bromoalcanoïque est injecté.

Après mélange des réactifs, l'étape i) consiste à laisser le milieu réactionnel sous agitation pendant une durée suffisante, généralement de 20 à 100 heures et de préférence 40 à 80 heures.

Selon un mode de réalisation, la réaction de l'étape i) est conduite en mode isotherme, c'est-à-dire avec une température réglée à une même valeur pendant toute la durée de la réaction.

Dans ce cas, l'étape i) est conduite à une température de 0 à 60°C. Selon un mode de réalisation, la température peut être de 0 à 5°C, ou de 5 à 10°C, ou de 10 à 15°C, ou de 15 à 20°C, ou de 20 à 25°C, ou de 25 à 30°C, ou de 30 à 35°C, ou de 35 à 40°C, ou de 40 à 45°C, ou de 45 à 50°C, ou de 50 à 55°C, ou encore de 55 à 60°C.

Selon un autre mode de réalisation, la réaction est conduite en mode de température croissante. Parmi les différents profils thermiques envisageables, il s'est avéré intéressant de prévoir des paliers à des températures croissantes, notamment allant d'une température initiale de 0 à 25°C jusqu'à une température finale de 26 à 60°C.

Selon une variante de réalisation, l'étape i) est conduite dans une batterie de réacteurs en parallèle R1, R2, ... Rn, 2 ≤ n ≤ 25, maintenus indépendamment à une température variable. Chaque réacteur dispose d'un programme de montée en température sur une durée donnée dépendante des températures choisies. Dans chaque réacteur, la température initiale du programme est préférentiellement comprise entre 0 et 25°C et la température finale de 26 à 60°C. Afin de maintenir une alimentation et un soutirage constant, régulier et continu en entrée et en sortie de la batterie de réacteurs, chaque réacteur fonctionne selon des cycles chargement/réaction/vidange avec un décalage de temps équivalent à la durée réactionnelle divisée par le nombre de réacteurs (n).

Dans toutes les variantes, la pression dans le ou les réacteurs, mesurée en point haut, généralement dans un ciel gazeux, est maintenue pendant l'étape i) au-dessus de la pression atmosphérique. En effet, cela permet le maintien d'une concentration élevée en ammoniac dans la phase liquide réactionnelle, laquelle favorise une sélectivité élevée en amine primaire. Il est ainsi possible de limiter la production d'impuretés, notamment d'amines secondaires voire tertiaires. On préfèrera néanmoins limiter la pression afin de limiter les coûts des réacteurs.

Aussi, la pression dans le ou les réacteurs est comprise entre 0.11 et 2.0 MPa absolu et de préférence 0.125 à 0.5 MPa absolu et de préférence 0.13 à 0.3 MPa absolu. Selon un mode de réalisation, la pression peut être de 0.11 à 0.15 MPa absolu, ou de 0.15 à 0.2 MPa absolu, ou 0.2 à 0.25 MPa absolu, ou de 0.25 à 0.3 MPa absolu, 0.3 à 0.35 MPa absolu, ou de 0.35 à 0.4 MPa absolu, 0.4 à 0.45 MPa absolu, ou de 0.45 à 0.5 MPa absolu, 0.5 à 0.55 MPa absolu, ou de 0.55 à 0.6 MPa absolu, 0.6 à 0.65 MPa absolu, ou de 0.65 à 0.7 MPa absolu, 0.7 à 0.75 MPa absolu, ou de 0.75 à 0.8 MPa absolu, 0.8 à 0.85 MPa absolu, ou de 0.85 à 0.9 MPa absolu, 0.9 à 0.95 MPa absolu, ou de 0.95 à 1.0 MPa absolu, ou de 1.0 à 1.05 MPa absolu, ou de 1.0 à 1.1 MPa absolu, ou de 1.10 à 1.15 MPa absolu, ou de 1.15 à 1.2 MPa absolu, ou 1.2 à 1.25 MPa absolu, ou de 1.25 à 1.3 MPa absolu, 1.3 à 1.35 MPa absolu, ou de 1.35 à 1.4 MPa absolu, 1.4 à 1.45 MPa absolu, ou de 1.45 à 1.5 MPa absolu, 1.5 à 1.55 MPa absolu, ou de 1.55 à 1.6 MPa absolu, 1.6 à 1.65 MPa absolu, ou de 1.65 à 1.7 MPa absolu, 1.7 à 1.75 MPa absolu, ou de 1.75 à 1.8 MPa absolu, 1.8 à 1.85 MPa absolu, ou de 1.85 à 1.9 MPa absolu, 1.9 à 1.95 MPa absolu, ou de 1.95 à 2.0 MPa absolu.

Lorsque l'on opère l'étape i) dans plusieurs réacteurs, les évents des réacteurs peuvent être connectés de façon à ce qu'ils fonctionnent à des pressions quasi identiques. Selon un autre mode de réalisation, il peut être avantageux de mettre les réacteurs sous une pression différente. Ainsi, lorsque la température est conduite de manière croissante de réacteur en réacteur comme expliqué plus haut, la pression dans les réacteurs peut être augmentée également de réacteur en réacteur. Selon un mode de réalisation, la pression régnant dans le ou les réacteurs est la pression autogène, imposée principalement par la tension de vapeur de l'ammoniac liée à la composition du milieu réactionnel et sa température.

Selon un mode de réalisation, on permet l'évaporation d'une partie de l'ammoniac afin de maintenir la température du milieu réactionnel. Ainsi, lorsque l'on est en mode continu, on peut refroidir le ou les premiers réacteurs par ce moyen. Lorsque l'on est en mode batch, on peut refroidir le réacteur par ce moyen en début de réaction. On peut soit réguler un débit d'évaporation d'ammoniac et dans ce cas on subit la pression, ou bien imposer une pression légèrement inférieure à l'équilibre liquide-vapeur et subir l'évaporation d'ammoniac. Avantageusement, entre 0,1 et 50%, et de préférence 10 à 30% de l'ammoniac injecté est évaporé au cours de l'étape i). L'homme de l'art saura adapter la quantité d'ammoniac à évaporer du milieu réactionnel pour le maintien du profil de température lors de la réaction. Ce mode de fonctionnement permet de limiter ou d'éviter les dispositifs d'échanges de chaleur coûteux tout en assurant le profil de température permettant des rendements optimaux.

Le procédé de l'invention permet ainsi de limiter fortement la quantité d'amine secondaire formée et d'augmenter par conséquent le rendement de la réaction.

Selon l'invention, le procédé comprend en outre une étape ii) de séparation de l'acide ω-aminoalcanoïque du mélange réactionnel à l'issue de l'étape i).

L'acide ω-aminoalcanoïque formé à l'issue de l'étape i) peut être séparé du mélange réactionnel de manière conventionnelle en soi, par exemple par les étapes suivantes.

Le mélange réactionnel issu de l'étape i) peut être dilué dans l'eau et chauffé à ébullition. L'ammoniac qui se dégage est abattu dans l'eau pour former une solution aqueuse d'ammoniac, laquelle peut être recyclée vers l'étape i) du présent procédé. Le mélange réactionnel appauvri en ammoniac peut être ensuite séparé de l'éventuelle couche d'huile formée par décantation à chaud avant d'être refroidi pour séparer l'acide ω-aminoalcanoïque par cristallisation et séparation solide liquide.

Par ailleurs, les filtrats riches en bromure d'ammonium récupérés de la séparation solide liquide peuvent être traités, par exemple à la soude, en vue de reformer de l'ammoniac qui est évaporé puis abattu dans l'eau et d'obtenir une solution aqueuse d'ammoniac.

L'acide ω-aminoalcanoïque peut également être séparé du mélange réactionnel obtenu à l'issue de l'étape i) par une étape de stripping afin d'enlever l'ammoniac qui est abattu dans l'eau, puis séparation solide/liquide telle une filtration sur filtre ou un essorage. Les eaux mères recueillies peuvent subir une extraction liquide-liquide, une cristallisation et/ou une filtration pour former une solution aqueuse riche en bromure d'ammonium et appauvrie en acide ω-aminoalcanoïque. Cette solution aqueuse riche en bromures d'ammonium peut être traitée à la soude pour reformer de l'ammoniac qui est évaporé puis abattu dans l'eau pour former une solution aqueuse d'ammoniac.

Le procédé de l'invention peut également comporter une étape iii) additionnelle, dans laquelle l'acide ω-aminoalcanoïque obtenu à l'étape ii) est soumis à un lavage et essorage. Le lavage peut notamment être réalisé à l'eau.

Le procédé de l'invention peut également comprendre en outre une étape iv) de purification de l'acide ω-aminoalcanoïque brut obtenu à l'issue de l'étape ii) ou iii).

Cette étape de purification peut notamment comprendre une ou plusieurs des opérations suivantes: recristallisation dans un solvant approprié comme l'eau ou une solution aqueuse basique ou acide telle qu'un mélange d'eau et d'acide carboxylique, filtration, lavage et essorage. De préférence, l'acide ω-aminoalcanoïque brut obtenu est purifié par recristallisation dans l'eau très chaude.

Aussi, le procédé de l'invention peut comprendre en outre une étape v) de récupération de l'acide ω-aminoalcanoïque résiduel dans les différents filtrats et eaux de lavage obtenues dans les étapes ii), iii) et/ou iv), notamment par dégazage de l'ammoniac, extraction liquide/liquide, cristallisation, filtration et lavage.

Enfin, le procédé de l'invention peut comprendre en outre une étape vi) de séchage de l'acide ω-aminoalcanoïque obtenu.

Avantageusement, le procédé selon l'invention permet d'obtenir un acide ω-aminoalcanoïque dans lequel le taux d'acide ω-aminodialcanoïque par rapport au taux d'acide ω-aminoalcanoïque est, à l'issue de l'étape i) et donc avant purification, inférieur à 1.8% massique, de préférence inférieur à 1.5% massique.

Par ailleurs, le procédé selon l'invention permet avantageusement une conversion complète de l'acide ω-bromoalcanoïque sur la durée de l'étape i) d'ammonolyse, dans un temps de réaction inférieur à 75h.

L'invention sera expliquée plus en détail dans les exemples qui suivent.

### [EXEMPLES]

### Exemple 1

Dans un autoclave d'un litre équipé d'une agitation mécanique à deux hélices à cinq pales et d'une turbine de type Rushton tournant à 400 rpm et d'un serpentin permettant la circulation d'un fluide caloporteur et d'un déverseur réglé à 0.15 MPa absolu, on place 660 g d'une solution aqueuse d'ammoniac à 40% massique refroidie à 0°C puis on ferme le réacteur. On ajoute par un goutte-à-goutte rapide 110 g d'acide 11-bromoundécanoïque (pureté 98%) fondu à 90°C puis on ajuste la température du milieu réactionnel à 22°C grâce au fluide caloporteur circulant dans le serpentin. La pression s'ajuste très rapidement à 0.15 MPa absolu et reste à cette valeur pendant toute la durée de la réaction. Après 12h30, on augmente la température du milieu réactionnel pour réaliser successivement des paliers de 12h30 respectivement à 24, 26, 28, 30 et 32°C. L'évent du réacteur est ensuite ouvert pour mettre le réacteur à pression atmosphérique.

Le milieu réactionnel est alors analysé. En dosant les ions bromure par du nitrate d'argent à l'aide d'une électrode d'argent, on constate que la totalité de l'acide 11-bromoundécanoïque a réagi. Par un dosage HPLC avec étalonnage externe, on mesure 1.3% massique d'acide 11-aminodiundécanoïque par rapport à l'acide 11-aminoundécanoïque.

### Exemple 2 (exemple de comparaison)

On reproduit l'exemple 1 mais en opérant à pression atmosphérique pendant toute la durée de l'expérience et en maîtrisant la vitesse d'ajout d'acide 11-bromoundécanoïque fondu pour que le milieu ne mousse pas de façon incontrôlée.

L'analyse du milieu réactionnel montre que la totalité de l'acide 11-bromoundécanoïque a réagi. On mesure 1.9% massique d'acide 11-aminodiundécanoïque par rapport à l'acide 11-aminoundécanoïque.

### Exemple 3 (exemple de comparaison)

On reproduit l'exemple 1, mais en opérant à pression atmosphérique pendant toute la durée de l'expérience et en utilisant 660 g d'une solution aqueuse d'ammoniac à 32% massique.

L'analyse du milieu réactionnel montre que la totalité de l'acide 11-bromoundécanoïque a réagi. On mesure 2.0% massique d'acide 11-amino-diundécanoïque par rapport à l'acide 11-aminoundécanoïque.

### [Liste des documents cités]

FR 988699
WO 2018/080869 A1
FR 928265
CN 103804209 B
EP 2 358 662 B1

## Revendications

1. Procédé de fabrication d'acide ω-aminoalcanoïque de formule NH₂-(CH₂)ₙ-COOH, dans laquelle n est un entier de 9 à 11, par réaction de l'acide ω-bromoalcanoïque correspondant avec l'ammoniac, comprenant les étapes suivantes:
i) réaction de l'acide ω-bromoalcanoïque avec une solution aqueuse d'ammoniac en excès, et
ii) séparation de l'acide ω-aminoalcanoïque formé du mélange réactionnel,
**caractérisé en ce que** la solution aqueuse d'ammoniac présente une concentration de 35 à 70% massique et que l'étape i) est conduite sous une pression supérieure à la pression atmosphérique, de 0.11 à 2.0 MPa absolu.

2. Procédé de fabrication selon la revendication 1, dans lequel l'étape i) est réalisée à une température comprise entre 0 et 60°C.

3. Procédé de fabrication selon la revendication 2, dans lequel l'étape i) est réalisée à une température constante.

4. Procédé de fabrication selon la revendication 2, dans lequel l'étape i) est réalisée à une température croissante.

5. Procédé de fabrication selon la revendication 4, dans lequel l'étape i) est réalisée à une température croissante, la température initiale allant de 0 à 25°C et la température finale allant de 26 à 60°C.

6. Procédé de fabrication selon l'une des revendications 1 à 5, dans lequel la solution aqueuse d'ammoniac présente une concentration de 36 à 60%, de préférence 38 à 45% massique.

7. Procédé de fabrication selon l'une des revendications 1 à 6, dans lequel le ratio massique entre l'acide ω-bromoalcanoïque au début de l'étape i) et la solution aqueuse d'ammoniac est de 1:3 à 1:20.

8. Procédé de fabrication selon l'une des revendications 1 à 7, dans lequel le procédé est conduit sous une pression de 0.125 à 0.5 MPa absolu.

9. Procédé de fabrication selon la revendication 8, dans lequel le procédé est conduit sous une pression de 0.13 à 0.3 MPa absolu.

10. Procédé de fabrication selon l'une des revendications 1 à 9, réalisé en mode continu.

11. Procédé de fabrication selon l'une des revendications 1 à 9, réalisé en mode batch.

12. Procédé selon l'une des revendications 1 à 11, comprenant en outre une étape iii) de lavage et essorage de l'acide ω-aminoalcanoïque obtenu à l'issue de l'étape ii).

13. Procédé selon la revendication 12 comprenant en outre une étape iv) de purification de l'acide ω-aminoalcanoïque brut obtenu à l'issue de l'étape ii) ou iii), de préférence par recristallisation dans l'eau bouillante.

14. Procédé selon la revendication 12 ou 13 comprenant en outre une étape v) de récupération de l'acide ω-aminoalcanoïque résiduel dans les différents filtrats et eaux de lavage obtenues dans les étapes ii), iii) et/ou iv), notamment par dégazage de l'ammoniac, extraction liquide/liquide, cristallisation, filtration et lavage.

15. Procédé selon l'une des revendications 1 et 14 comprenant en outre une étape vi) de séchage de l'acide ω-aminoalcanoïque obtenu.

## Patentansprüche

1. Verfahren zur Herstellung von ω-Aminoalkansäure mit der Formel NH₂-(CH₂)ₙ-COOH, wobei n eine ganze Zahl von 9 bis 11 ist, durch Umsetzung der entsprechenden ω-Bromalkansäure mit Ammoniak, wobei es die folgenden Schritte umfasst:
i) Umsetzen von ω-Bromalkansäure mit einer wässrigen, im Überschuss vorliegenden Ammoniaklösung, und
ii) Abtrennen der ω-Aminoalkansäure, die sich gebildet hat, von der Reaktionsmischung,
**dadurch gekennzeichnet, dass** die wässrige Ammoniaklösung eine Konzentration von 35 bis 70 Massen-% hat und dass der Schritt i) unter einem Druck durchgeführt wird, welcher um 0,11 bis 2,0 MPa absolut höher als der Atmosphärendruck ist.

2. Herstellungsverfahren nach Anspruch 1, wobei der Schritt i) bei einer Temperatur im Bereich von 0 bis 60 °C durchgeführt wird.

3. Herstellungsverfahren nach Anspruch 2, wobei der Schritt i) bei einer konstanten Temperatur durchgeführt wird.

4. Herstellungsverfahren nach Anspruch 2, wobei der Schritt i) bei einer ansteigenden Temperatur durchgeführt wird.

5. Herstellungsverfahren nach Anspruch 4, wobei der Schritt i) bei einer ansteigenden Temperatur durchgeführt wird, wobei die Anfangstemperatur im Bereich von 0 bis 25 °C liegt und die Endtemperatur im Bereich von 26 bis 60 °C liegt.

6. Herstellungsverfahren nach einem der Ansprüche 1 bis 5, wobei die wässrige Ammoniaklösung eine Konzentration von 36 bis 60 %, vorzugsweise 38 bis 45 % nach Masse aufweist.

7. Herstellungsverfahren nach einem der Ansprüche 1 bis 6, wobei die wässrige Ammoniaklösung eine Konzentration von 36 bis 60 %, vorzugsweise 38 bis 45 % nach Masse aufweist.

8. Herstellungsverfahren nach einem der Ansprüche 1 bis 7, wobei das Verfahren bei einem Druck von 0,125 bis 0,5 MPa absolut durchgeführt wird.

9. Herstellungsverfahren nach einem der Ansprüche 1 bis 8, wobei das Verfahren bei einem Druck von 0,13 bis 0,3 MPa absolut durchgeführt wird.

10. Herstellungsverfahren nach einem der Ansprüche 1 bis 9, wobei es kontinuierlich durchgeführt wird.

11. Herstellungsverfahren nach einem der Ansprüche 1 bis 9, wobei es chargenweise durchgeführt wird.

12. Verfahren nach einem der Ansprüche 1 bis 11, wobei es darüber hinaus einen Schritt iii) des Waschens und des Entfernens flüssiger Stoffe aus der ω-Aminoalkansäure umfasst, welche nach Abschluss des Schrittes ii) erhalten wurde.

13. Verfahren nach Anspruch 12, wobei es darüber hinaus einen Schritt iv) des Aufreinigens der rohen ω-Aminoalkansäure umfasst, welche nach Abschluss des Schrittes ii) oder iii) erhalten wurde, vorzugsweise durch Umkristallisierung in siedendem Wasser.

14. Verfahren nach Anspruch 12 oder 13, wobei es darüber hinaus einen Schritt v) des Gewinnens der w-Aminoalkansäure umfasst, welche in den verschiedenen Filtraten und Waschwässern verbleibt, wie in den Schritten ii), iii) und/oder iv) anfallen, insbesondere durch Austreiben des Ammoniaks, flüssig/flüssig-Extraktion, Kristallisation, Filtration und Waschen.

15. Verfahren nach einem der Ansprüche 1 und 14, wobei es darüber hinaus einen Schritt vi) des Trocknens der erhaltenen ω-Aminoalkansäure umfasst.

## Claims

1. Process for the manufacture of w-aminoalkanoic acid of formula NH₂-(CH₂)ₙ-COOH, in which n is an integer from 9 to 11, by reaction of the corresponding ω-bromoalkanoic acid with ammonia, comprising the following stages:
i) reaction of the ω-bromoalkanoic acid with an aqueous excess ammonia solution, and
ii) separation of the w-aminoalkanoic acid formed from the reaction mixture,
**characterized in that** the aqueous ammonia solution exhibits a concentration of 35% to 70% by weight and that stage i) is carried out under a pressure greater than atmospheric pressure, from 0.11 to 2.0 MPa absolute.

2. Manufacturing process according to Claim 1, in which stage i) is carried out at a temperature of between 0 and 60°C.

3. Manufacturing process according to Claim 2, in which stage i) is carried out at a constant temperature.

4. Manufacturing process according to Claim 2, in which stage i) is carried out at an increasing temperature.

5. Manufacturing process according to Claim 4, in which stage i) is carried out at an increasing temperature, the initial temperature ranging from 0 to 25°C and the final temperature ranging from 26 to 60°C.

6. Manufacturing process according to one of Claims 1 to 5, in which the aqueous ammonia solution exhibits a concentration of 36% to 60%, preferably of 38% to 45%, by weight.

7. Manufacturing process according to one of Claims 1 to 6, in which the ratio by weight of the ω-bromoalkanoic acid at the start of stage i) to the aqueous ammonia solution is from 1:3 to 1:20.

8. Manufacturing process according to one of Claims 1 to 7, in which the process is carried out under a pressure of 0.125 to 0.5 MPa absolute.

9. Manufacturing process according to Claim 8, in which the process is carried out under a pressure of 0.13 to 0.3 MPa absolute.

10. Manufacturing process according to one of Claims 1 to 9, carried out continuously.

11. Manufacturing process according to one of Claims 1 to 9, carried out batchwise.

12. Process according to one of Claims 1 to 11, additionally comprising a stage iii) of washing and draining the w-aminoalkanoic acid obtained on conclusion of stage ii).

13. Process according to Claim 12, additionally comprising a stage iv) of purification of the crude ω-aminoalkanoic acid obtained on conclusion of stage ii) or iii), preferably by recrystallization from boiling water.

14. Process according to Claim 12 or 13, additionally comprising a stage v) of recovery of the residual ω-aminoalkanoic acid in the various filtrates and aqueous washing liquors obtained in stages ii), iii) and/or iv), in particular by degassing the ammonia, liquid-liquid extraction, crystallization, filtration and washing.

15. Process according to one of Claims 1 and 14, additionally comprising a stage vi) of drying the ω-aminoalkanoic acid obtained.
